# FASCICULE DE BREVET EUROPEEN

(11) **EP 2 764 363 B1**
(45) Date de publication et mention de la délivrance du brevet: **14.02.2018**
(21) Numéro de dépôt: 12779109.3
(22) Date de dépôt: 03.10.2012
(51) Int. Cl.: A61J 1/05, A61B 5/145, A61B 5/15, G01N 33/558, A61B 5/151, A61B 5/157, B01L 3/00

(54) **ENSEMBLE POUR DETERMINER LA PRESENCE OU L'ABSENCE D'ANALYTE DANS UN ECHANTILLON DE SANG ET UNITE D'ANALYSE COMPRENANT UN TEL ENSEMBLE**
ANORDNUNG ZUR BESTIMMUNG DER AN-ODER ABWESENHEIT EINES ANALYTEN IN EINER BLUTPROBE UND ANALYSEVORRICHTUNG MIT EINER SOLCHEN ANORDNUNG
ASSEMBLY FOR DETERMINING THE PRESENCE OR ABSENCE OF AN ANALYTE IN A BLOOD SAMPLE AND ANALYSIS UNIT COMPRISING SUCH AN ASSEMBLY

(30) Priorité: 05.10.2011 FR 1158998
(43) Date de publication de la demande: 13.08.2014
(60) Demande divisionnaire: 17198188.9
(73) Titulaire: Biomérieux, 69280 Marcy l'Étoile (FR)
(72) Inventeur: COLIN, Bruno, F-69280 Marcy L'Etoile (FR); PARIS, Cécile, F-69690 Bessenay (FR); GOUDARD, Michel, F-69290 Saint Genis Les Ollieres (FR)
(74) Mandataire: Chevalier, Renaud Philippe
(86) Numéro de dépôt international: PCT/FR2012/052237
(87) Numéro de publication internationale: WO 2013/050701

(56) Documents cités:
- WO-A1-03/049609
- WO-A1-2010/064998
- WO-A2-2008/075213
- US-A1- 2010 160 831

## Description

La présente invention concerne un ensemble pour réaliser un essai dit test rapide en vue de déterminer la présence ou l'absence d'au moins un analyte dans un échantillon de sang humain ou animal. De plus, la présente invention concerne une unité d'analyse comprenant un tel ensemble. La présente invention trouve notamment application dans le domaine des analyses cliniques ou industrielles.

Les tests rapides ou dosages à flux latéralsont couramment utilisés dans les domaines d'analyses cliniques, alimentaires, pharmaceutiques et chimiques pour déterminer la présence ou l'absence de divers analytes, tels que des anticorps, des antigènes, des hormones, des protéines, des molécules chimiques dans des échantillons liquides, tels que des échantilons de sang. Les tests rapides sont commercialisés soit sous la forme de bandelettes simples comprenant tous les éléments physiques et biologiques nécessaires à l'analyse, soit sous la forme de cassette dans laquelle est incluse la bandelette du dosage.

A titre d'exemple la demande de brevet US2006172435A1 décrit un test rapide pour réaliser un essai en vue de déterminer la présence ou l'absence d'un analyte dans un échantillon de sang humain ou animal. Le test rapide du document US2006172435A1 comprend un boîtier transportable à la main et une bandelette qui est fixée au boîtier et qui présente une zone d'application de l'échantillon de sang. La bandelette comprend un réactif adapté pour réagir en présence de l'analyte, de façon à modifier l'aspect visuel d'une zone test de la bandelette. Les documents WO 03/049609, WO 2010/064998, WO 2008/075213 et US 2010160831 décrivent également de tels tests rapides sans toutefois divulguer la présence d'un récipient calibré et adapté pour prélever, contenir et restituer un échantillon de sang. En pratique pour réaliser un essai dit test rapide, il faut réaliser plusieurs actions préalables dans des conditions d'hygiène et de sécurité : désinfection locale de la zone de prélèvement, mise à disposition de l'élément de piquage conservé dans des conditions stériles, piquage du patient ou de l'animal pour prélever l'échantillon à analyser, dépôt de l'échantillon au niveau de la bandelette d'analyse dans une zone appropriée dénommée zone d'application de l'échantillon.

Ainsi, comme décrit dans la demande de brevet précitée l'utilisateur met en oeuvre une aiguille, pour percer la peau et un vaisseau sanguin, et un récipient, pour contenir l'échantillon de sang afin de l'appliquer sur la zone d'application.

Cependant, les inconvénients liés aux pratiques de l'art antérieur sont multiples :
- l'utilisateur doit se procurer :
- le test rapide proprement dit,
- les éléments de piquage et de prélèvement de l'échantillon, et
- le récipient de collecte de l'échantillon,
ce qui nécessite plusieurs emballages distincts pour chaque élément précité ; de plus, chacun des éléments précités doit être parfaitement calibré en fonction de l'analyse à effectuer, et
de plus l'utilisateur doit se procurer un moyen indépendant de stérilisation locale de la zone de piquage chez le patient ou l'animal.

D'une manière générale, un test rapide de l'art antérieur nécessite que l'utilisateur s'assure des bonnes pratiques d'hygiène et de sécurité. Or, dans certains contextes d'organisations déficientes, d'économies précaires etc..., ces bonnes pratiques d'hygiène et de sécurité peuvent ne pas être respectées, ce qui peut induire des risques importants de contamination croisée.

La présente invention vise notamment à résoudre les problèmes mentionnés ci-avant.

A cet effet, l'invention a pour objet un ensemble, pour réaliser un essai en vue de déterminer la présence ou l'absence d'au moins un analyte dans un échantillon de sang humain ou animal, l'ensemble comprenant au moins :
- un support transportable à la main ;
- une bandelette qui est fixée au support et qui comprend une zone d'application de l'échantillon de sang et au moins un réactif nécessaire à l'analyse ;
- un élément de piquage, lié au support et adapté pour percer la peau et un vaisseau sanguin ; l'ensemble (1) étant caractérisé en ce qu'il comprend en outre un récipient calibré et adapté pour prélever, contenir et restituer l'échantillon de sang, l'élément de piquage étant inséré totalement ou partiellement dans le récipient, ledit récipient étant lié au support de manière amovible de sorte que le récipient peut être disposé alternativement dans une configuration de rangement et dans une configuration d'utilisation, dans laquelle le récipient peut être placé près de la zone d'application.

En d'autres termes, le support, la bandelette, l'élément de piquage et le récipient sont fixés ou liés entre eux de façon à former un ensemble autonome.

Selon un mode de réalisation de l'invention, l'ensemble comprend en outre une pièce textile qui est imprégnée de produit désinfectant et qui s'étend sur une surface externe du support, de préférence sur la surface opposée à la zone d'application, l'ensemble comprenant en outre une pellicule scellée, détachable et étanche, la pellicule étant agencée de façon à recouvrir la pièce textile en configuration de rangement.

Dans un mode de réalisation préférentiel, la pièce textile est repliée au niveau de la surface externe du support opposée à la zone d'application.

Ainsi, une telle pièce textile permet à l'utilisateur, avant et après la piqûre, de désinfecter la peau à l'emplacement de la piqûre et la pointe le cas échéant. En position repliée, la pièce textile représente un encombrement négligeable.

Ainsi, un tel ensemble permet de regrouper tous les composants nécessaires à l'essai d'analyse, en particulier leur conditionnement et leur transport peut être réalisé au moyen d'un emballage unique. De plus, la liaison entre l'élément de piquage et le support évite la perte de l'aiguille et améliore la manipulation de l'ensemble. Enfin, un tel ensemble permet de répondre à toutes les exigences d'hygiène et de sécurité, d'éviter les problèmes de contaminations croisées et d'assurer un prélèvement sanguin dont le volume est parfaitement calibré.

Dans un mode de réalisation de l'invention, l'élément de piquage stérile est obtenu par surmoulage et reste stérile jusqu'à son utilisation, en particulier quand le récipient n'est pas détaché du support. En d'autres termes, l'élément de piquage est stérilisé lors de son surmoulage, à plus de 120°C voire plus de 150°C, par la matière plastique formant le récipient, c'est-à-dire ses parois et/ou son embase.

Ainsi, l'élément de piquage est stérilisé lors de la fabrication de l'ensemble, ce qui évite de prévoir une étape de stérilisation supplémentaire.

Dans la présente demande, le terme « ensemble » désigne un groupe de composants qui sont nécessaires pour réaliser un essai en vue de déterminer la présence ou l'absence d'au moins un analyte dans un échantillon de sang humain ou animal.

Dans la présente demande, le terme « lier » et ses dérivés désignent une liaison matérielle ou mécanique, directe ou indirecte, entre deux éléments. En d'autres termes, deux éléments peuvent être mécaniquement liés entre eux ou par l'intermédiaire d'un troisième élément.

Dans la présente demande, le terme « élément de piquage » désigne une pointe, une aiguille, une lame ou tout organe équivalent adapté pour percer la peau et un vaisseau sanguin. Dans la présente demande, le terme « percer » et ses dérivés désigne les actions consistant à perforer ou à couper.

Dans la présente demande, le terme « récipient » désigne un objet creux capable de contenir un liquide, par exemple un échantillon sanguin. En d'autres termes, le récipient définit globalement une cavité dans laquelle peut être contenu le liquide.

Dans la présente demande, l'expression « calibré et adapté pour prélever, contenir et restituer l'échantillon de sang humain ou animal » indique que l'utilisateur peut prélever et contenir un volume de sang adéquat et nécessaire à l'analyse en plaçant le récipient près de la piqûre, puis restituer ce volume sur la zone d'application de la bandelette.

Dans la présente demande, le terme « inséré » et ses dérivés désignent l'insertion de l'élément de piquage dans le récipient, c'est-à-dire soit à l'intérieur du volume creux défini par le récipient, soit enfiché dans le récipient (c'est-à-dire sur une embase ou une paroi de ce récipient).

Selon un mode de réalisation de l'invention, en configuration de rangement, le récipient est fixé au support et l'élément de piquage est fixé au support.

En d'autres termes, le récipient est lié directement au support et l'élément de piquage est lié directement au support.

Selon un autre mode de réalisation de l'invention, en configuration de rangement, le récipient entoure tout ou partie de l'élément de piquage, le récipient étant totalement ou partiellement inséré dans le support.

Ainsi, un tel agencement permet de réaliser un ensemble compact en configuration de rangement, car l'élément de piquage est logé dans le volume du récipient.

Selon un autre mode de réalisation de l'invention, en configuration de rangement, le récipient est lié au support par l'intermédiaire d'un moyen de compression, retenu par un moyen de blocage, ledit moyen de compression étant également en liaison avec l'élément de piquage.

Un tel moyen de compression peut être formé par un moyen de rappel élastique, tel qu'un ressort. Ainsi, un tel moyen de compression et un tel moyen de blocage permettent de manipuler aisément le récipient.

Selon une variante de l'invention, l'élément de piquage est mobile entre une configuration de rangement, dans laquelle l'élément de piquage est disposé à l'intérieur du support, et une configuration de piquage, dans laquelle l'élément de piquage est agencé en saillie sur un bord du support.

Ainsi, un tel élément de piquage mobile permet de former un ensemble sûr et compact, car l'élément de piquage n'est pas disposé en saillie sur un bord du support ; et car un utilisateur ne risque pas une piqûre inopinée.

Selon une variante de l'invention, l'élément de piquage est mobile entre la configuration de rangement, dans laquelle l'élément de piquage est situé à l'intérieur du support, en retrait dans le support ou affleurant à une surface externe du support, et la configuration d'utilisation, dans laquelle l'élément de piquage est sorti partiellement en saillie par rapport à une surface externe du support.

Selon une autre variante de l'invention, l'élément de piquage est mobile entre la configuration de rangement, dans laquelle l'élément de piquage est en retrait à l'intérieur du support, et la configuration d'utilisation, dans laquelle l'élément de piquage est sorti partiellement en saillie par rapport au support par libération sous l'action par détente d'un organe de compression.

Un tel organe de compression peut être formé par un organe de rappel élastique, tel qu'un ressort.

Selon une variante de l'invention, l'ensemble comprend un bouton de commande agencé sur le support de façon à libérer l'organe de compression. Le bouton de commande peut par exemple coopérer par encliquetage élastique avec l'organe de compression.

Selon un mode de réalisation de l'invention, en configuration de rangement, le récipient est lié au support par l'intermédiaire de l'élément de piquage, l'élément de piquage étant fixé au support, l'ensemble comprenant une embase qui est enfichée sur l'élément de piquage, le récipient étant fixé sur l'embase.

Ainsi, une telle embase permet de protéger un utilisateur, par exemple le personnel médical, d'une piqûre inopinée, car l'élément de piquage est enfiché sur l'embase. De plus, une telle embase permet à l'utilisateur de manipuler facilement le récipient.

Selon un mode de réalisation de l'invention, l'embase est formée par un capuchon agencé pour obturer une extrémité du récipient, le capuchon étant réalisé en un matériau résilient de façon à pouvoir être enfiché sur l'élément de piquage.

Ainsi, un tel capuchon permet d'obturer le récipient et de fixer de manière rapide et amovible l'embase, donc le récipient, sur l'élément de piquage.

Selon un mode de réalisation de l'invention, le support est formé par un boîtier, tel qu'une cassette globalement parallélépipédique, et dans lequel la bandelette, de préférence poreuse, est disposée à l'intérieur du boîtier, le boîtier présentant au moins une ouverture pour dégager la zone d'application et au moins une ouverture pour visualiser le résultat de l'essai au niveau d'une zone test.

Ainsi, un tel boîtier et une telle bandelette permettent de réaliser des essais pour la plupart des analytes à rechercher.

Selon une variante de l'invention, le boîtier est formé de deux demi-coques comprenant des moyens de fixation d'une demi-coque sur l'autre demi-coque.

Ainsi, un tel boîtier permet un montage facile de la bandelette dans une demi-coque et il assure une étanchéité efficace du volume intérieur où est montée la bandelette.

Selon un mode de réalisation de l'invention, l'élément de piquage est agencé en saillie sur un bord du support.

Ainsi, un tel agencement permet d'utiliser l'élément de piquage directement, ce qui évite de manipuler l'ensemble pour sortir l'élément de piquage.

Selon un mode de réalisation de l'invention, l'élément de piquage est formé par une aiguille ou par une lame.

Ainsi, une telle aiguille permet de perforer la peau et un vaisseau sanguin, ce qui peut être réalisé par un utilisateur sans qualifications, par exemple par la personne sur lequel l'échantillon.

Selon un mode de réalisation de l'invention, le récipient a globalement la forme d'un tube, de préférence à base circulaire.

Ainsi, un tel récipient est simple à manipuler et à fabriquer et il est bien adapté pour recueillir un échantillon de sang.

Selon un mode de réalisation de l'invention, le récipient est composé d'un matériau translucide, de préférence transparent.

Ainsi, un tel récipient permet de visualiser le volume de sang, donc de prélever juste le volume nécessaire.

Selon un mode de réalisation de l'invention, le récipient a un volume compris entre 30 microlitres et 200 microlitres, de préférence entre 60 microlitres et 100 microlitres.

Ainsi, un tel volume permet de réaliser des essais pour la plupart des analytes à rechercher.

Selon une variante de l'invention, le récipient peut comporter une marque de niveau.

Ainsi, une telle marque permet d'indiquer à l'utilisateur quant le volume prélevé est suffisant pour déterminer la présence ou l'absence de l'analyte.

Selon une variante de l'invention, l'ensemble comprend en outre une portion rigide faisant saillie sur un bord du support, de manière à permettre le détachement de la pellicule détachable.

Ainsi, une telle portion rigide permet de détacher facilement la pellicule détachable.

Par ailleurs, la présente invention a pour objet une unité d'analyse, pour réaliser un essai en vue de déterminer la présence ou l'absence d'au moins un analyte dans un échantillon de sang humain ou animal, l'unité d'analyse comprenant un ensemble selon l'invention et un emballage stérile, l'ensemble étant disposé dans l'emballage stérile.

La présente invention sera bien comprise et ses avantages ressortiront aussi à la lumière de la description qui va suivre, donnée uniquement à titre d'exemple non limitatif et faite en référence aux dessins annexés, dans lesquels :
- la figure 1 est une vue de face d'un ensemble conforme à l'invention, en configuration de rangement ;
- la figure 2 est une vue en perspective de l'ensemble de la figure 1 ;
- la figure 3 est une vue similaire à la figure 2 de l'ensemble de la figure 2 en configuration d'utilisation ;
- la figure 4 est une vue du détail IV à la figure 3 ;
- la figure 5 est une vue en perspective, suivant un angle différent de la figure 2, de l'ensemble de la figure 2, en configuration ;
- la figure 6 est une vue similaire à la figure 5 de l'ensemble de la figure 5, en configuration d'utilisation ;
- la figure 7 est une vue d'une partie de l'ensemble de la figure 1, à l'état désassemblé ;
- la figure 8 est une vue similaire à la figure 3 d'une partie d'un ensemble conforme à une variante de l'invention, en configuration de rangement ; et
- la figure 9 est une vue de l'ensemble de la figure 8 en configuration d'utilisation.

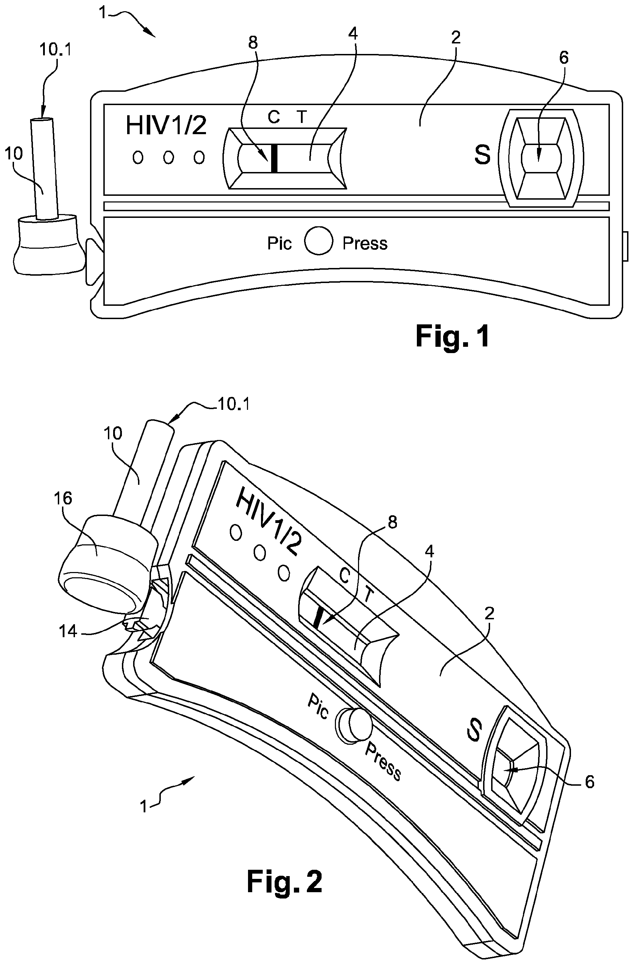

Les figures 1 et 2 illustrent un ensemble 1 qui a notamment pour fonction de permettre un essai en vue de déterminer la présence ou l'absence d'au moins un analyte dans un échantillon de sang humain ou animal.

L'ensemble 1 comprend un support qui est ici défini par un boîtier 2 et qui est transportable à la main, par exemple par un utilisateur, car son encombrement est faible. Dans l'exemple des figures 1 et 2, le boîtier 2 forme une cassette globalement parallélépipédique mesurant environ 70 mm x 35 mm x 6 mm.

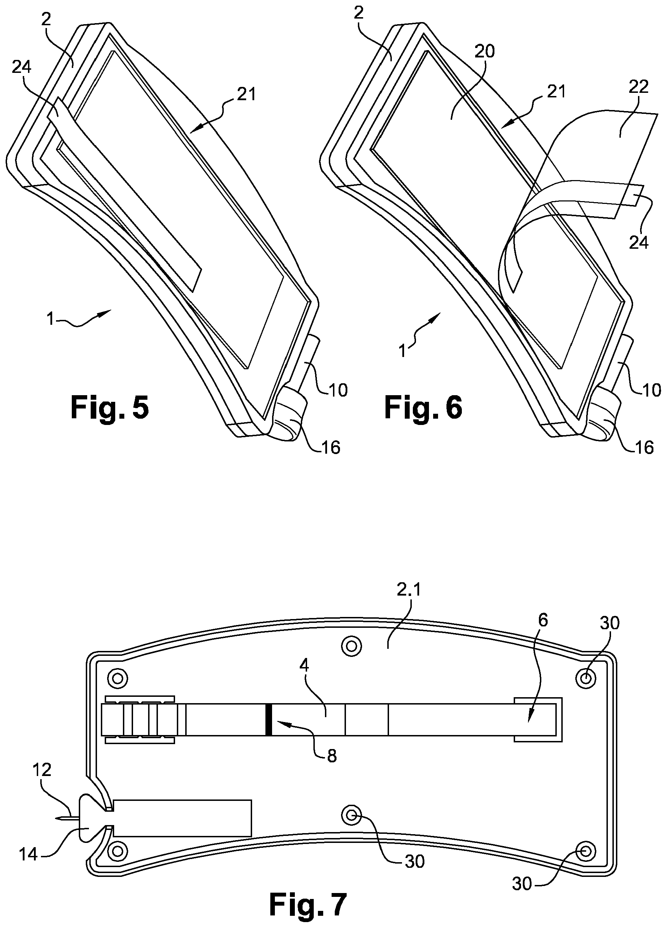

En outre, l'ensemble 1 comprend une bandelette 4, pour l'essai d'analyse. La bandelette 4 est fixée au boîtier 2, comme cela est décrit ci-après en relation avec la figure 7. La bandelette 4 comprend une zone d'application 6 où doit être appliquée l'échantillon de sang. La bandelette 4 est réalisée en un matériau poreux. La bandelette 4 comprend en outre au moins un réactif nécessaire à l'analyse. Un tel réactif est adapté pour réagir en présence de l'analyte, de façon à faire apparaître le résultat de l'essai au niveau d'une zone test 8 de la bandelette 4.

L'ensemble 1 comprend en outre un récipient 10 calibré et adapté pour prélever, contenir et restituer l'échantillon de sang humain ou animal. À cet effet, le récipient 10 présente une ouverture 10.1. De plus, le récipient a un volume d'environ 75 microlitres. Dans l'exemple des figures 1 et 2, le récipient 10 a globalement la forme d'un tube à base circulaire. Le récipient 10 est composé d'un matériau translucide, en l'occurrence d'un matériau transparent.

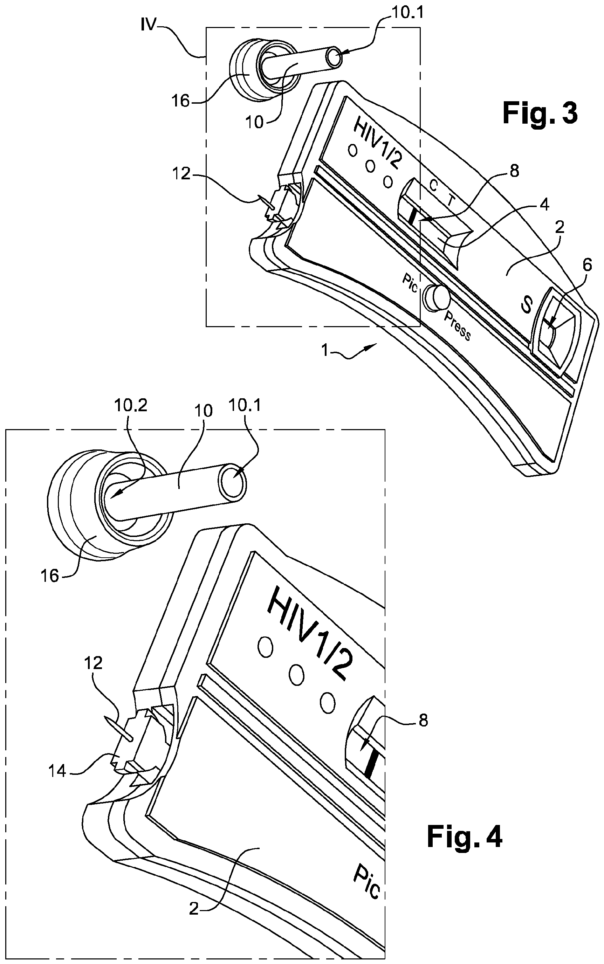

Comme le montrent les figures 3 et 4, l'ensemble 1 comprend en outre un l'élément de piquage qui est ici définie par une aiguille 12 et qui est adaptée pour percer la peau et un vaisseau sanguin. À cet effet, l'aiguille 12 présente une extrémité perforante. L'aiguille 12 peut être par exemple réalisée en un acier inoxydable.

De plus, le récipient 10 est lié au boîtier 2 de manière amovible de sorte que le récipient 10 peut être disposé alternativement dans une configuration de rangement (figures 1 et 2) et dans une configuration d'utilisation (figures 3 et 4), dans laquelle le récipient 12 peut être placé près de la zone d'application 6.

L'aiguille 12 est liée au boîtier 2. Dans l'exemple des figures 3 et 4, l'aiguille 12 est solidaire d'un porte-aiguille 14 qui appartient au boîtier 2. L'aiguille 12 est agencée en saillie sur un bord du boîtier 2, en configuration de rangement (figures 1 et 2) et en configuration d'utilisation (figures 3 et 4). L'aiguille 12 est fixe ou immobile par rapport au boîtier 2.

En configuration de rangement (figures 1 et 2), le récipient 10 est lié au boîtier 2 par l'intermédiaire de l'aiguille 12. En configuration de rangement (figures 1 et 2), le récipient 10 est donc immobile par rapport au boîtier 2. À cet effet, l'aiguille 12 est fixée au boîtier 2. L'ensemble 1 comprend en outre une embase pour lier indirectement le récipient 10 au boîtier 2. L'embase est ici définie par un capuchon 16.

Le capuchon 16 est fixé au récipient 10, par exemple par de la colle et le capuchon 16 est enfiché sur l'aiguille 12. Ainsi, l'élément de piquage 12 est inséré (planté) dans le capuchon 16 qui appartient au récipient 10.

Le capuchon 16 est agencé pour obturer une extrémité 10.2 du récipient 10. Dans l'exemple des figures 1 à 4, le capuchon 16 est réalisé en un matériau ductile de sorte que le capuchon 16 peut être enfiché sur l'aiguille 12.

Comme le montrent les figures 5 et 6; l'ensemble 1 comprend en outre une pièce textile 20 qui est imprégnée de produit désinfectant. La pièce textile 20 est ici formée par une lingette en matériau non tissé. La pièce textile 20 s'étend sur la face arrière 21 du boîtier 2, c'est-à-dire sur la surface externe opposée à la zone d'application 6.

Comme le montre la figure 6, l'ensemble 1 comprend en outre une pellicule détachable 22 qui est agencée de façon à recouvrir la pièce textile 20. La pellicule détachable 22 est étanche, notamment au produit désinfectant. La pellicule détachable 22 est ici réalisée en un matériau plastique de synthèse. Dans l'exemple de la figure 6, la pièce textile 20 est repliée au niveau de la surface externe 21 du support 2 opposée à la zone d'application 6.

Comme le montre la figure 7, la bandelette 4 est disposée à l'intérieur du boîtier 2. En pratique, la bandelette 4. Le boîtier 2 présente une ouverture pour dégager la zone d'application 6 et une ouverture pour visualiser le résultat de l'essai au niveau d'une zone test 8.

L'ensemble 1 comprend en outre une portion rigide 24 qui fait saillie sur un bord du boîtier 2, de manière à permettre le détachement de la pellicule détachable 22, donc le dégagement de la pièce textile 20. La portion rigide 24 peut être formée par une bande en matériau synthétique ou métallique. La portion rigide 24 peut être disposée entre deux strates de la pellicule détachable 22. La portion rigide 24 peut avantageusement comporter une indication intelligible, par écrit ou par idéogramme, pour indiquer à un utilisateur comment détacher la pellicule détachable 22.

Comme le montre la figure 7, le boîtier 2 est formé de deux demi-coques, dont l'une porte la référence 2.1 à la figure 7. Les demi-coques 2.1 et équivalent délimitent un volume creux dans lequel sont logées la bandelette 4 et une partie de l'aiguille 12.

Chaque demi-coque 2.1 et équivalent comprend des moyens de fixation d'une demi-coque sur l'autre demi-coque, de façon à assembler le boîtier 2. Dans l'exemple de la figure 7, la demi-coque 2.1 comprend des parties femelles d'encliquetage 30 pour l'encliquetage élastique de parties mâles non représentées appartenant à la demi-coque non représentée.

Comme le montre la figure 7, l'aiguille 12 est obtenue par surmoulage et elle reste stérile jusqu'à son utilisation, en particulier quand le récipient 10 n'est pas détaché du support 2.

En service, un utilisateur dispose d'une unité d'analyse conforme à l'invention non représentée. L'utilisateur sort l'ensemble 1 de son emballage stérile non représenté. Pour former une unité d'analyse conforme à l'invention, l'ensemble 1 est emballé dans un environnement stérile.

Ensuite, l'utilisateur tire sur le récipient 10 et le capuchon 16, de façon à les ôter de l'aiguille 12. L'utilisateur peut poser le récipient 10 et le capuchon 16 sur une table à côté du boîtier 2.

Puis, l'utilisateur tire la portion rigide 24 pour détacher totalement ou partiellement la pellicule détachable 22. L'utilisateur peut accéder à la pièce textile 20 ainsi dégagée.

L'utilisateur désinfecte la peau à l'emplacement de la piqûre et l'aiguille 12 le cas échéant.

L'utilisateur perce la peau et un vaisseau sanguin à l'aide de l'aiguille 12, puis prélève, dans le récipient 10, un volume de sang adéquat et nécessaire à l'analyse.

Ensuite, l'utilisateur place le récipient 10 près de la zone d'application 6 de façon à y verser ou restitué l'échantillon de sang prélevé.

Alors, l'échantillon de sang diffuse ou migre dans la bandelette 4 jusqu'au réactif, qui réagit si l'analyte est présent dans l'échantillon de sang diffusé.

Le cas échéant, l'utilisateur peut visualiser le résultat de l'essai au niveau de la zone test 8, en fonction de la présence ou de l'absence d'analyte dans l'échantillon de sang recueilli.

Ainsi, l'utilisateur peut déterminer si le test est positif ou négatif.

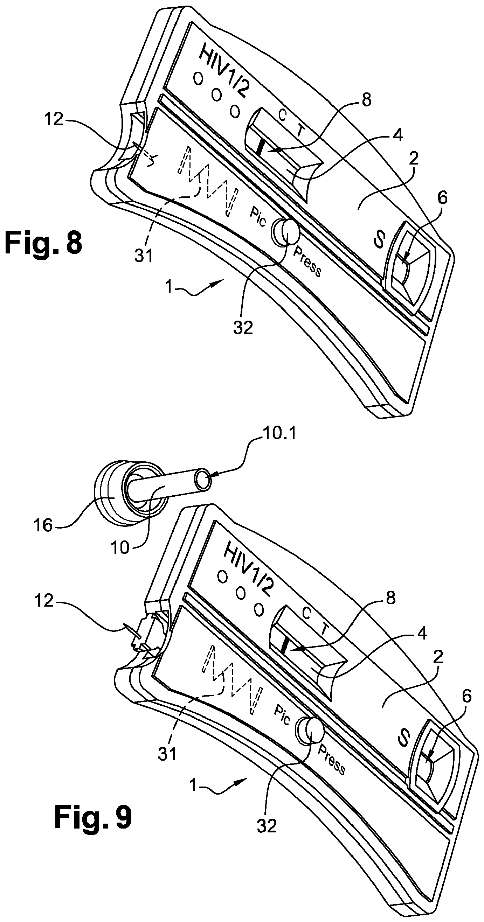

Les figures 8 et 9 illustrent un ensemble 1 conforme à une variante de l'invention. Dans la mesure où l'ensemble 1 des figures 8 et 9 est similaire à l'ensemble 1 des figures 1 à 7, la description de l'ensemble 1 donnée ci-avant en relation avec les figures 1 à 7 peut être transposée à l'ensemble 1 des figures 8 et 9, à l'exception des différences notables énoncées ci-après.

L'ensemble 1 diffère de l'ensemble 1, notamment car, dans l'ensemble 1, l'élément de piquage 12 est mobile entre la configuration de rangement (figure 8), dans laquelle l'élément de piquage 12 est situé à l'intérieur du support 2, et la configuration d'utilisation (figure 9), dans laquelle l'élément de piquage 12 est sorti partiellement en saillie par rapport à une surface externe du support 2. Dans l'exemple des figures 8 et 9, l'élément de piquage 12 est disposé affleure à une surface externe du support 2.

De plus, l'ensemble 1 diffère de l'ensemble 1, car pour déplacer l'élément de piquage 12 entre la configuration de rangement (figure 8) et la configuration d'utilisation (figure 9), l'ensemble 1 comprend en outre un organe de rappel élastique en compression, ici symbolisé par un ressort hélicoïdal 31, dont la détente libère l'élément de piquage 12.

En outre, l'ensemble 1 comprend un bouton de commande 32 qui est agencé sur le support 2 de façon à libérer l'organe de compression (ressort hélicoïdal 31). Le bouton de commande 32 coopère par exemple par encliquetage élastique avec l'organe de compression (ressort hélicoïdal 31).

En configuration de rangement, comme dans l'exemple des figures 1 à 7, le récipient 10 est lié au support par l'intermédiaire de l'élément de piquage 12, l'élément de piquage étant en liaison coulissante avec le support 2, l'ensemble 1 comprenant une embase (capuchon 16) qui est enfichée, par surmoulage, sur l'élément de piquage 12, le récipient 10 étant fixé sur l'embase (capuchon 16).

Lorsque l'utilisateur presse le bouton de commande 32, l'encliquetage élastique libère le ressort hélicoïdal 31, ce qui permet de déplacer l'élément de piquage 12 vers la configuration d'utilisation (figure 9).

Selon d'autres caractéristiques avantageuses mais facultatives, prises isolément ou selon toute combinaison techniquement admissible :
- Le récipient est fixé au support et l'élément de piquage est fixé au support, ce qui réalise une liaison directe entre le récipient et le support, alternativement à la liaison indirecte entre le récipient 10 et le boîtier 2 décrite ci-avant.
- En configuration de rangement, le récipient peut entourer tout ou partie de l'élément de piquage, le récipient étant totalement ou partiellement inséré dans le support.

## Revendications

1. Ensemble (1), pour réaliser un essai en vue de déterminer la présence ou l'absence d'au moins un analyte dans un échantillon de sang humain ou animal, l'ensemble (1) comprenant au moins :
- un support (2) transportable à la main ;
- une bandelette (4) qui est fixée au support (2) et qui comprend une zone d'application (6) de l'échantillon de sang et au moins un réactif nécessaire à l'analyse ;
- un élément de piquage (12) adapté pour percer la peau et un vaisseau sanguin ;
l'ensemble (1) étant **caractérisé en ce qu'**il comprend en outre un récipient (10) calibré et adapté pour prélever, contenir et restituer l'échantillon de sang, l'élément de piquage (12) étant inséré totalement ou partiellement dans le récipient (10), ledit récipient (10) étant lié au support (2) de manière amovible de sorte que le récipient (10) peut être disposé alternativement dans une configuration de rangement et dans une configuration d'utilisation, dans laquelle le récipient (10) peut être placé près de la zone d'application (6).

2. Ensemble (1) selon la revendication 1, comprenant en outre une pièce textile (20) qui est imprégnée de produit désinfectant et qui s'étend sur une surface externe (21) du support (2), l'ensemble (1) comprenant en outre une pellicule (22) scellée, détachable et étanche, la pellicule (22) étant agencée de façon à recouvrir la pièce textile (20) en configuration de rangement.

3. Ensemble (1) selon la revendication 2, dans lequel la pièce textile (20) est repliée au niveau de la surface externe (21) du support (2) opposée à la zone d'application (6).

4. Ensemble selon l'une des revendications précédentes, dans lequel, en configuration de rangement, le récipient est fixé au support, et dans lequel l'élément de piquage est fixé au support.

5. Ensemble selon la revendication 4, dans lequel, en configuration de rangement, le récipient entoure tout ou partie de l'élément de piquage, le récipient étant totalement ou partiellement inséré dans le support.

6. Ensemble selon l'une des revendications précédentes, dans lequel, en configuration de rangement, le récipient est lié au support par l'intermédiaire d'un moyen de compression retenu par un moyen de blocage, ledit moyen de compression étant également en liaison avec l'élément de piquage.

7. Ensemble (1) selon l'une des revendications précédentes, dans lequel, en configuration de rangement, le récipient (10) est lié au support (2) par l'intermédiaire de l'élément de piquage (12), l'élément de piquage (12) étant fixé au support (2), l'ensemble (1) comprenant une embase qui est enfichée sur l'élément de piquage (12), le récipient (10) étant fixé sur l'embase.

8. Ensemble (1) selon la revendication 7, dans lequel l'embase est formée par un capuchon (16) agencé pour obturer une extrémité (10.1) du récipient (10), le capuchon étant réalisé en un matériau résilient de façon à pouvoir être enfiché sur l'élément de piquage (12).

9. Ensemble (1) selon l'une des revendications précédentes, dans lequel le support est formé par un boîtier (2), tel qu'une cassette globalement parallélépipédique, et dans lequel la bandelette (4) est disposée à l'intérieur du boîtier (2), le boîtier (2) présentant au moins une ouverture pour dégager la zone d'application (6) et au moins une ouverture pour visualiser le résultat de l'essai au niveau d'une zone test (8).

10. Ensemble (1) selon l'une des revendications précédentes, dans lequel l'élément de piquage (12) est agencé en saillie sur un bord du support (2).

11. Ensemble (1) selon l'une des revendications précédentes, dans lequel l'élément de piquage (12) est formé par une aiguille ou par une lame.

12. Ensemble (1) selon l'une des revendications précédentes, dans lequel le récipient (10) a globalement la forme d'un tube.

13. Ensemble (1) selon l'une des revendications précédentes, dans lequel le récipient (10) est composé d'un matériau translucide.

14. Ensemble (1) selon l'une des revendications précédentes, dans lequel le récipient (10) a un volume compris entre 30 microlitres et 200 microlitres.

15. Unité d'analyse, pour réaliser un essai en vue de déterminer la présence ou l'absence d'au moins un analyte dans un échantillon de sang humain ou animal, l'unité d'analyse comprenant un ensemble selon l'une des revendications précédentes et un emballage stérile, l'ensemble étant disposé dans l'emballage stérile.

16. Ensemble (1), pour réaliser un essai en vue de déterminer la présence ou l'absence d'au moins un analyte dans un échantillon de sang humain ou animal, l'ensemble (1) comprenant au moins :
- un support (2) transportable à la main ;
- une bandelette (4) qui est fixée au support (2) et qui comprend une zone d'application (6) de l'échantillon de sang et au moins un réactif nécessaire à l'analyse ;
- un élément de piquage (12) adapté pour percer la peau et un vaisseau sanguin ;
l'ensemble (1) étant **caractérisé en ce que** l'élément de piquage (12) est lié au support (2) et **en ce qu'**il comprend en outre un récipient (10) calibré et adapté pour prélever, contenir et restituer un échantillon de sang, le récipient (10) étant lié au support (2) de manière amovible de sorte que le récipient (10) peut être disposé alternativement dans une configuration de rangement et dans une configuration d'utilisation, dans laquelle le récipient (10) peut être placé près de la zone d'application (6).

## Patentansprüche

1. Einheit (1) zum Ausführen eines Tests zum Bestimmen der Gegenwart oder der Abwesenheit mindestens eines Analyten in einer menschlichen oder tierischen Blutprobe, wobei die Einheit (1) mindestens umfasst:
- einen von Hand transportierbaren Träger (2);
- einen Streifen (4), der an dem Träger (2) befestigt ist und eine Auftragezone (6) der Blutprobe und mindestens ein Reagens, das für die Analyse erforderlich ist, umfasst;
- ein Stechelement (12), das angepasst ist, um die Haut und ein Blutgefäß zu durchstoßen;
wobei die Einheit (1) **dadurch gekennzeichnet ist, dass** sie außerdem einen Behälter (10) umfasst, der kalibriert und angepasst ist, um die Blutprobe zu entnehmen, zu enthalten und zurückzugeben, wobei das Stechelement (12) vollständig oder teilweise in den Behälter (10) eingefügt ist, wobei der Behälter (10) mit dem Träger (2) abnehmbar derart verbunden ist, dass der Behälter (10) alternativ in einer Verstaukonfiguration und in einer Gebrauchskonfiguration, in der der Behälter (10) nahe der Auftragezone (6) platziert werden kann, angeordnet sein kann.

2. Einheit (1) nach Anspruch 1, die außerdem ein Textilteil (20) umfasst, das mit einem Desinfektionsprodukt getränkt ist und sich auf einer äußeren Oberfläche (21) des Trägers (2) erstreckt, wobei die Einheit (1) außerdem ein versiegeltes, abnehmbares und dichtes Häutchen (22) umfasst, wobei das Häutchen (22) derart eingerichtet ist, dass es das Textilteil (20) in Verstaukonfiguration abdeckt.

3. Einheit (1) nach Anspruch 2, wobei das Textilteil (20) im Bereich der äußeren Oberfläche (21) des Trägers (2), die der Auftragezone (6) entgegengesetzt ist, umgefaltet ist.

4. Einheit nach einem der vorhergehenden Ansprüche, wobei der Behälter in Verstaukonfiguration an dem Träger befestigt ist, und wobei das Stechelement an dem Träger befestigt ist.

5. Einheit nach Anspruch 4, wobei der Behälter in Verstaukonfiguration das Stechelement ganz oder teilweise umgibt, wobei der Behälter vollständig oder teilweise in den Träger eingefügt ist.

6. Einheit nach einem der vorhergehenden Ansprüche, wobei der Behälter in Verstaukonfiguration mit dem Träger über ein Kompressionsmittel verbunden ist, das durch ein Blockierungsmittel zurückgehalten wird, wobei das Kompressionsmittel auch mit dem Stechelement in Verbindung steht.

7. Einheit (1) nach einem der vorhergehenden Ansprüche, wobei der Behälter (10) in Verstaukonfiguration mit dem Träger (2) über das Stechelement (12) verbunden ist, wobei das Stechelement (12) an dem Träger (2) befestigt ist, wobei die Einheit (1) einen Sockel umfasst, der auf dem Stechelement (12) angesteckt ist, wobei der Behälter (10) auf dem Sockel befestigt ist.

8. Einheit (1) nach Anspruch 7, wobei der Sockel aus einer Kappe (16) gebildet ist, die eingerichtet ist, um ein Ende (10.1) des Behälters (10) zu verschließen, wobei die Kappe aus einem federnden Werkstoff derart hergestellt ist, dass sie auf dem Strichelement (12) angesteckt werden kann.

9. Einheit (1) nach einem der vorhergehenden Ansprüche, wobei der Träger durch ein Gehäuse (2), wie zum Beispiel eine insgesamt parallelepipedische Kassette, gebildet ist, und wobei der Streifen (4) in dem Inneren des Gehäuses (2) angeordnet ist, wobei das Gehäuse (2) mindestens eine Öffnung zum Freilegen der Auftragezone (6) und mindestens eine Öffnung zum Anzeigen des Resultats des Tests im Bereich der Testzone (8) aufweist.

10. Einheit (1) nach einem der vorhergehenden Ansprüche, wobei das Stechelement (12) auf einem Rand des Trägers (2) vorstehend eingerichtet ist.

11. Einheit (1) nach einem der vorhergehenden Ansprüche, wobei das Stechelement (12) aus einer Nadel oder einer Klinge gebildet ist.

12. Einheit (1) nach einem der vorhergehenden Ansprüche, wobei der Behälter (10) insgesamt die Form einer Röhre hat.

13. Einheit (1) nach einem der vorhergehenden Ansprüche, wobei der Behälter (10) aus einem durchsichtigen Werkstoff besteht.

14. Einheit (1) nach einem der vorhergehenden Ansprüche, wobei der Behälter (10) ein Volumen zwischen 30 Mikroliter und 200 Mikroliter hat.

15. Analyseeinheit zum Ausführen eines Tests zum Bestimmen der Gegenwart oder Abwesenheit mindestens eines Analyten in einer menschlichen oder tierischen Blutprobe, wobei die Analyseeinheit eine Einheit nach einem der vorhergehenden Ansprüche und eine sterile Verpackung umfasst, wobei die Einheit in der sterilen Verpackung angeordnet ist.

16. Einheit (1) zum Ausführen eines Tests zum Bestimmen der Gegenwart oder Abwesenheit mindestens eines Analyten in einer menschlichen oder tierischen Blutprobe, wobei die Einheit (1) mindestens umfasst:
- einen von Hand transportierbaren Träger (2);
- einen Streifen (4), der an dem Träger (2) befestigt ist und eine Auftragezone (6) der Blutprobe und mindestens ein Reagens, das für die Analyse erforderlich ist, umfasst;
- ein Stechelement (12), das angepasst ist, um die Haut und ein Blutgefäß zu durchstoßen; wobei die Einheit (1) **dadurch gekennzeichnet ist, dass** das Stechelement (12) mit dem Träger (2) verbunden ist, und dadurch, dass sie außerdem einen Behälter (10) umfasst, der kalibriert und angepasst ist, um eine Blutprobe zu entnehmen, zu enthalten und zurückzugeben, wobei der Behälter (10) mit dem Träger (2) abnehmbar derart verbunden ist, dass der Behälter (10) alternativ in einer Verstaukonfiguration und in einer Gebrauchskonfiguration, in der der Behälter (10) nahe der Auftragezone (6) platziert werden kann, angeordnet sein kann.

## Claims

1. An assembly (1), to perform an assay for determining the presence or absence of at least one analyte in a human or animal blood sample, the assembly (1) comprising at least:
- a manually transportable support (2);
- a strip (4) which is fastened to the support (2) and which comprises an application area (6) of the blood sample and at least one reagent required for the analysis;
- a pricking element (12) adapted to pierce the skin and a blood vessel; the assembly (1) being **characterized in that** it further comprises a calibrated container (10) adapted to take, contain and restore the blood sample, the pricking element (12) being inserted totally or partially into the container (10), said container (10) being removably linked to the support (2) so that the container (10) can be disposed alternately in a stowed configuration and in a use configuration, in which the container (10) can be placed close to the application area (6).

2. The assembly (1) according to claim 1, further comprising a textile part (20) which is impregnated with disinfectant and which extends on an outer surface (21) of the support (2), the assembly (1) further comprising an sealed, detachable and airtight film (22), the film (22) being arranged so as to cover the textile part (20) in a stowed configuration.

3. The assembly (1) according to claim 2, wherein the textile part (20) is folded at the outer surface (21) of the support (2) opposite the application area (6).

4. The assembly according to any of the preceding claims, wherein, in the stowed configuration, the container is fastened to the support, and in which the pricking element is fastened to the support.

5. The assembly according to claim 4, wherein, in the stowed configuration, the container surrounds all or part of the pricking element, the container being totally or partially inserted into the support.

6. The assembly according to any of the preceding claims, wherein, in the stowed configuration, the container is linked to the support by means of a compression means retained by a blocking means, said compression means being also in connection with the pricking element.

7. The assembly (1) according to any of the preceding claims, wherein in the stowed configuration, the container (10) is linked to the support (2) by means of the pricking element (12), the pricking element (12) being fastened to the support (2), the assembly (1) comprising a base which is plugged into the pricking element (12), the container (10) being fastened on the base.

8. The assembly (1) according to claim 7, wherein the base is formed by a cap (16) arranged to obturate one end (10.1) of the container (10), the cap being made of resilient material so that it can be plugged onto the pricking element (12).

9. The assembly (1) according to any of the preceding claims, wherein the support is formed by a casing (2), such as a generally parallelepipedic cassette, and in which the strip (4) is disposed inside the casing (2), the casing (2) having at least one opening for disengaging the application area (6) and at least one opening for displaying the result of the assay at a test area (8).

10. The assembly (1) according to any of the preceding claims, wherein the pricking element (12) is arranged protruding on an edge of the support (2).

11. The assembly (1) according to any of the preceding claims, wherein the pricking element (12) is formed by a needle or a blade.

12. The assembly (1) according to any of the preceding claims, wherein the container (10) has the overall shape of a tube.

13. The assembly (1) according to any of the preceding claims, wherein the container (10) is made of a translucent material.

14. The assembly (1) according to any of the preceding claims, wherein the container (10) has a volume comprised between 30 microliters and 200 microliters.

15. An analysis unit, to perform an assay for determining the presence or absence of at least one analyte in a human or animal blood sample, the analysis unit comprising an assembly according to any of the preceding claims and a sterile package, the assembly being disposed in the sterile package.

16. The assembly (1), to perform an assay for determining the presence or absence of at least one analyte in a human or animal blood sample, the assembly (1) comprising at least:
- a manually transportable support (2);
- a strip (4) which is fastened to the support (2) and which comprises an application area (6) of the blood sample and at least one reagent required for the analysis;
- a pricking element (12) adapted to pierce the skin and a blood vessel; the assembly (1) being **characterized in that** the pricking element (12) is linked to the support (2) and **in that** it further comprises a calibrated container (10), adapted to take, contain and restore a blood sample, the container (10) being removably linked to the carrier (2) so that the container (10) can be disposed alternately in a stowed configuration and in a use configuration, in which the container (10) can be placed close to the application area (6).
